# NEUE EUROPÄISCHE PATENTSCHRIFT

(11) **EP 1 702 633 B2**
(45) Veröffentlichungstag und Bekanntmachung des Hinweises auf die Entscheidung über den Einspruch: **29.06.2016**
(45) Hinweis auf die Patenterteilung: 16.01.2013
(21) Anmeldenummer: 06003911.2
(22) Anmeldetag: 27.02.2006
(51) Int. Cl.: A61K 6/083, A61L 27/14, A61L 27/50, A61L 24/00

(54) **Hochschlagzähe (high impact) Prothesenkunststoffe**
High impact plastics for prosthesis
Matériaux plastiques pour prothèses résistants au choc

(30) Priorität: 17.03.2005 DE 102005012825
(43) Veröffentlichungstag der Anmeldung: 20.09.2006
(73) Patentinhaber: Heraeus Kulzer GmbH, 63450 Hanau (DE)
(72) Erfinder: Renz, Karl-Heinz, 71131 Jettingen (DE); Stange, Frank, 88085 Langenargen (DE); Koschalka, Oliver, 63543 Neuberg (DE); Erdrich, Albert, Dr., 61231 Bad Nauheim (DE); Grundler, Andreas, Dr., 42117 Wuppertal (DE)
(74) Vertreter: Bendele, Tanja

(56) Entgegenhaltungen:
- WO-A2-02//058592
- GB-A- 1 584 530
- US- - 3 347 954

## Beschreibung

Die Erfindung betrifft High Impact Prothesenkunststoffe und Mischungen zu deren Herstellung.

Zur Herstellung von zahntechnischen Prothesen (Totalprothesen, Teilprothesen, Zahnspangen, etc.) für das Tragen im Mund sind verschiedene Materialien erhältlich:
1. Heißpolymerisierende Kunststoffe (1 oder 2 Komponenten). Diese weisen einen sehr hohen thermisch bedingten Volumenschrumpf auf, der zu Passungenauigkeiten führt.
2. Autopolymerisierende Kunststoffe (2 Komponenten).
3. Lichtpolymerisierende Kunststoffe (1 oder 2 Komponenten).
4. Thermoplastische Kunststoffe (1 Komponente), solche Materialien sind zahntechnisch relativ schwer zu verarbeiten.
5. Mikrowellenhärtende Kunststoffe (1 oder 2 Komponenten). Auch hier führt ein sehr hoher thermisch bedingter Volumenschrumpf führt zu Passungenauigkeiten Außerdem können die aus den unter 1. bis 5. genannten Materialien gefertigten Prothesen beim Herunterfallen oder sonstiger unachtsamer Behandlung leicht brechen. Diese unerwünschten Eigenschaften werden durch Verwendung sogenannter High Impact¹ Kunststoffe beseitigt:
6. Heißpolymerisierende High Impact Kunststoffe (1 oder 2 Komponenten). Diese weisen allerdings wiederum den unerwünscht hohen thermisch bedingten Volumenschrumpf auf, der zu Passungenauigkeiten führt.
¹ Der Begriff High-Impact wird in der ISO 1567 - Denture Base Materials näher erläutert. Danach ist der Prothesenkunststoff dann ein High-Impact-Denture-Base-Material, wenn er in der Schlagzähigkeit nach ISO 1567 (an Charpy angelehnt) einen Wert von 2 kJ/m² übersteigt.

Auf dem Markt ist allerdings die Unterscheidung zwischen High Impact Material und anderen Kunststoffen nicht einheitlich geregelt, da die Messmethode ungenügend ist. Zur Bestimmung des High-Impact-Charakters wird daher demnächst ein neues Prüfverfahren (Bruchzähigkeit) zur Anwendung kommen. Die Bruchzähigkeitsmessung ist angelehnt an die ASTM E 399-90 und wurde für Prothesenkunststoffe modifiziert. Das Einfügen der Methode in die ISO 1567 als Ersatz für die Charpy-Schlagzähigkeit ist beschlossen und steht kurz bevor.

DE 199 41 829 beschreibt autopolymerisierbare Dentalzusammesetzungen, die Perlpolymerisate enthalten und z.B. Prothesenbasismaterialien ergeben.

In DE 196 17 876 A1 werden Dentalmaterialien mit Polysiloxan Schlagfestigkeitsmodifikatoren beschrieben. Sie weisen nach Härtung eine verbesserte Schlagfestigkeit auf.

US 5,182,332 beschreibt Dentalzusammensetzungen mit gepfropften, Kautschuk enthaltenden Copolymeren.

GB 1584530 A offenbart (die Verweise in Klammern beziehen sich auf dieses Dokument): heißhärtende Dentalmaterialien mit verbesserten mechanischen Eigenschaften (Beschreibung Seite 1, Zeile 5-6), die durch ein Flüssigkeit/Pulver Verfahren hergestellt sind. Das durch eine elastische Polyurethanphase modifizierte Perlpolymerisat ist Polymethylmethacrylat (Ansprüche 1-10). Das Perlpolymerisat ist mit einer Monomerlösung gemischt, die Monomerlösung enthält auch einen Initiator und andere Additive.

Es ist nun gelungen, einen autopolymerisierenden Prothesenkunststoff zu entwickeln, der die Eigenschaften eines High-Impact-Prothesenkunststoffes zeigt und dabei den Nachteil des hohen thermisch bedingten Volumenschrumpfs nicht aufweist. Der Kunststoff entspricht überraschenderweise der künftigen ISO Norm 1567.

Die Ausgangsmischungen für Prothesenkunststoffe bestehen in der Regel aus einer Monomer- und einer Pulverkomponente, die vor der Anwendung gemischt werden.

Die High Impact Eigenschaft wird erreicht, indem in der Pulverkomponente anstelle eines herkömmlichen Perlpolymerisats ein durch eine elastische Phase modifiziertes Perlpolymerisat verwendet wird.

Die Erfindung betrifft somit die Verwendung nach Anspruch 1.

Gegenstand der Erfindung ist die Verwendung mindestens eines durch eine elastische Phase modifizierten Perlpolymerisats anstelle eines herkömmlichen Perlpolymerisats,
wobei die elastische Phase aus
I. Poly-(n-butyl-acrylat) PBA
II. Butadien-Styrol-Copolymer
ausgewählt ist, und
wobei die Verteilung der elastischen Phase in der Pulverkomponente einer der Möglichkeiten a bis c folgt, nämlich
a) elastischer Kern in fester Schale (Kern-Schale-Teilchen); oder
b) mehrere elastische Kerne in einer festen Matrix; oder
c) Kern-Schale Teilchen aus a), in fester Matrix verteilt;
wobei die elastische Phase einen Durchmesser zwischen 10 nm und 100 µm besitzt,
in Komponente B eines autopolymerisierenden 2-Komponenten-Prothesenbasismaterials bestehend aus
A) einer flüssigen Monomerkomponente,
B) einer pulverförmigen füllstoffhaltigen Komponente,
C) ein Initiatorsystem, wobei das Initiatorsystem eine Kombination von Barbitursäuren in Verbindung mit Kupfer- und Chloridionen und Benzoyl- oder Laurylperoxid ist,
zur Erzielung einer
Bruchzähigkeit von größer gleich 2 MPa m^{1/2} und einer
Brucharbeit von größer gleich 900 J/m² in einem High Impact Prothesenkunststoff.

Komponente A und/oder B) enthalten vorzugsweise zusätzlich Stoffe aus den Gruppen der Füllstoffe, Pigmente, Stabilisatoren und Regler.

Die Erfindung betrifft auch den gehärteten Kunststoff, der die künftige ISO Norm 1567erfüllt, nämlich einen High Impact Prothesenkunststoff mit einer Bruchzähigkeit von ≥ 2 MPa · m^{1/2} und einer Brucharbeit von ≥ 900 J/m², der mindestens ein durch eine elastische Phase modifiziertes Perlpolymerisat enthält.

Die Modifikation des Perlpolymerisats erfolgtdurch folgende elastische Materialien:
2. Butadien-Styrol-Coploymer (Literatur: J Dent 1986; 14; 214-217 bzw. US 3,427,274)
3. Poly (n-butyl-acrylat) PBA (Literatur: Polymer Volume 39 Number 14 1998 3073-3081).

Die Verteilung der elastischen Phase in der festen Matrix kann folgendermaßen aussehen:
a) Elastischer Kern in fester Schale (Kern-Schale-Teilchen);
b) mehrere elastische Kerne in fester Matrix;
c) Kern-Schale Teilchen aus a), in fester Matrix verteilt;
d) elastische und feste Phase bilden interpenetrierende Netzwerke.

Zu a), b) und c): Die elastische Phase besitzt hierbei einen Durchmesser zwischen 10 nm und 100 µm, vorzugsweise zwischen 60 und 5000 nm.

Als Monomere kommen die auf dem Dentalgebiet üblichen Monomere in Betracht: Beispiele sind radikalisch polymerisierbare monofunktionelle Monomere wie Mono(meth)acrylate, Methyl-, Ethyl-, Butyl-, Benzyl-, Furfuryl- oder Phenyl(meth)acrylat, di- bzw. polyfunktionelle Monomere wie di- oder polyfunktionelle Acrylate bzw. Methacrylate, z.B. Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Hexamethylendiisocyanat), Di-, Tri- oder Tetraethylenglykoldi(meth)acrylat, Decandioldi(meth)acrylat, Dodecandioldi(meth)acrylat, Hexyldecandioldi(meth)acrylat, Trimethylolpropantri(meth)acrylat, Pentaerythrittetra(meth)-acrylat sowie Butandioldi(meth)acrylat, Ethylenglycol-di(meth)acrylat, Polyethylenglycol-di(meth)acrylate, ethoxylierte/propoxylierte Bisphenol-A-di(meth)acrylate.

Als Füllstoffe kommen z.B. pyrogene oder Fällungskieselsäuren, Dentalgläser wie Aluminosilicatgläser oder Fluoroaluminosilicatgläser, Strontiumsilicat, Strontiumborosilicat, Lithiumsilicat, Lithiumaluminiumsilicat, Schichtsilikate, Zeolithe, amorphe sphärische Füller auf Oxid- oder Mischoxidbasis (SiO₂, ZrO₂ und/oder TiO₂), Metalloxide mit Primärteilchengröße von ca. 40 bis 300 nm, Splitterpolymerisate mit 10 - 100 µm Teilchengröße (vgl. R. Janda, Kunststoffverbundsysteme, VCH Verlagsgesellschaft, Weinheim, 1990, Seite 225 ff.) oder deren Mischungen in Frage. Zudem können Verstärkungsmittel wie Glasfasern, Polyamid- oder Kohlenstoffasern eingearbeitet werden.

Die Füllstoffe werden in der Regel zu 0 bis 80 Gew.%, vorzugsweise zu 0 bis 3 Gew.%, bezogen auf die gesamte Prothesenkunststoffzusammensetzung bzw. die Summe der Komponenten A und B, eingesetzt.

Als Regler zur Einstellung des Molekulargewichts kommen z.B. in Frage:
TGEH: Thioglykolsäure-2-ethylhexylester
t-DDM: tert.-Dodecylmercaptan
GDMA: Glykoldimercaptoacetat.

Geeignete Stabilisatoren sind z. B. Hydrochinonmonomethylether oder 2,6-Di-tert.-butyl-4-methylphenol (BHT).

Des weiteren können die erfindungsgemäßen Prothesenbasismaterialien weitere übliche Zusatzstoffe enthalten, z.B. aus den Gruppen der antimikrobiellen Additive, UV-Absorber, Thixotropiermittel, Katalysatoren und Vernetzer.

Solche Additive werden - wie die Pigmente, Stabilisatoren und Regler - in eher geringen Mengen eingesetzt, insgesamt 0,01 bis 3,0, besonders 0,01 bis 1,0 Gew.% bezogen auf die Gesamtmasse des Materials.

Die Aushärtung der Zusammensetzungen erfolgt durch redoxinduzierte radikalische Polymerisation bei Raumtemperatur bzw. bei leicht erhöhter Temperatur unter leichtem Druck, um Blasenbildung zu vermeiden.

Als Initiatoren für die bei Raumtemperatur durchgeführte Polymerisation werden Redoxinitiator-Kombinationen gemäss Anspruch 1 eingesetzt. Das beanspruchte Initiatorsystem ist eine Kombination von Barbitursäuren in Verbindung mit Kupfer- und Chlorid-ionen sowie Benzoyl- oder Laurylperoxid. Dieses System zeichnet sich durch eine hohe Farbstabilität aus.

Die Materialien der Erfindung werden bevorzugt im Dentalbereich angewandt, vor allem zur Herstellung von Prothesen oder kieferorthopädische Apparaturen zur Korrektur der Zahnstellung. Weitere Anwendungsmöglichkeiten ergeben sich aber in allen Bereichen, in denen ein hochschlagzäher Formkörper individuell zu erstellen ist, z.B. bei
- Knochenzementen mit verbesserter Schlagzähigkeit
- veterinärmedizinischen Anwendungen, in denen die Schlagzähigkeit hoch sein muss, z.B. Hufreparaturmaterial oder Zahnersatz für Tiere.

### Beispiel:

Das folgende Beispiel erläutert die Erfindung näher:

### Erfindungsgemäße Zusammensetzung:

Eine Monomermischung bestehend aus
Methylmethacrylat 93,85%, Butandioldimethacrylat 6%, Trioctylmethylammoniumchlorid 0,15%, Kupfer(II)chlorid-dihydrat 10ppm
und eine Pulverkomponente bestehend aus
Polymethylmethacrylat 30% (z.B. Plexidon M449 der Fa. Röhm GmbH), durch eine elastische Phase modifiziertes Perlpolymerisat 67,8% (z.B. DA 441 der Fa. MV Plastics Ltd.), 1-Benzyl-5-phenylbarbitursäure 0,9%, 5-n-Butylbarbitursäure 0,3%, Dibenzoylperoxid 1%
werden angemischt, in eine Gussform gebracht und bei 55 °C 30 min lang polymerisiert.

### Vergleichszusammensetzung:

Eine Monomermischung bestehend aus
Methylmethacrylat 93,85%, Butandioldimethacrylat 6%, Trioctylmethylammoniumchlorid 0,15%, Kupfer(II)chlorid-dihydrat 10ppm
und eine Pulverkomponente bestehend aus
Polymethylmethacrylat 20% (z.B. Plexidon M449 der Fa. Röhm GmbH), Polymethylmethacrylat-Copolymer 78,8%, 1-Benzyl-5-phenylbarbitursäure 0,9%, 5-n-Butylbarbitursäure 0,3%, Dibenzoylperoxid 0,8%
werden angemischt, in eine Gussform gebracht und bei 55 °C 30 min lang polymerisiert.

Aus dem gehärteten Kunststoff werden Probenkörper geschnitten und in Anlehnung an ASTM E 399-90, modifiziert für Prothesenkunststoffe, vermessen.

Es ergeben sich folgende mechanischen Prüfwerte:

| | Vergleich | Erfindung |
|---|---|---|
| | herkömml. Perlpolymerisat | modif. Perlpolymerisat |
| Bruchzähigkeit | 1,4 MPa x m^{1/2} | 2,1 MPa x m^{1/2} |
| Brucharbeit | 183 J/m² | 1026 J/m² |

### Versuchsergebnis:

Die Werte der erfindungsgemäßen Zusammensetzung sind deutlich höher als die des bisher verwendeten, herkömmlichen Autopolymerisats. Folglich ist das Material mechanisch stabiler und wird auch die neuen Mindestvorgaben der ISO für High-Impact-Kunststoffe (2 MPa x m^{1/2} und 900 J/m²) erfüllen.

## Patentansprüche

1. Verwendung
mindestens eines durch eine elastische Phase modifizierten Perlpolymerisats anstelle eines herkömmlichen Perlpolymerisats,
wobei die elastische Phase aus
I. Poly-(n-butyl-acrylat) PBA
II. Butadien-Styrol-Copolymer
ausgewählt ist, und
wobei die Verteilung der elastischen Phase in der Pulverkomponente einer der Möglichkeiten a bis c folgt, nämlich
a) elastischer Kern in fester Schale (Kern-Schale-Teilchen); oder
b) mehrere elastische Kerne in einer festen Matrix; oder
c) Kern-Schale Teilchen aus a), in fester Matrix verteilt;
wobei die elastische Phase einen Durchmesser zwischen 10 nm und 100 µm besitzt,
in Komponente B eines autopolymerisierenden 2-Komponenten-Prothesenbasismaterials bestehend aus
A) einer flüssigen Monomerkomponente,
B) einer pulverförmigen füllstoffhaltigen Komponente,
C) ein Initiatorsystem, wobei das Initiatorsystem eine Kombination von Barbitursäuren in Verbindung mit Kupfer- und Chloridionen und Benzoyl- oder Laurylperoxid ist,
zur Erzielung einer
Bruchzähigkeit von größer gleich 2 MPa m^{1/2} und einer
Brucharbeit von größer gleich 900 J/m²
in einem High Impact Prothesenkunststoff.

## Claims

1. Use of at least one bead polymer modified through an elastic phase instead of a conventional bead polymer,
whereby the elastic phase is selected from
I poly-(n-butyl-acrylate) PBA
II butadiene-styrene copolymer
and whereby the distribution of the elastic phase in the powder component corresponds to one of options a to c, namely
a) elastic core in a solid shell (core-shell particle) or
b) multiple elastic cores in a solid matrix or
c) core-shell particle from a), distributed in a solid matrix,
whereby the elastic phase has a diameter between 10 nm and 100 µm
in component B of an autopolymerizing 2-component denture base material consisting of
A) a liquid monomer component,
B) a powder-like filler-containing component,
C) an initiator system, wherein the initiator system is a combination of barbituric acids in conjunction with copper- and chloride-ions and benzoyl- or lauryl-peroxide,to attain a fracture toughness of ≥ 2 MPa m^{½} and a fracture work of ≥ 900 J/m² in a high-impact denture plastic material.

## Revendications

1. Utilisation d'au moins un polymère en perles modifié par une phase élastique à la place d'un polymère en perles traditionnel,
dans laquelle la phase élastique est sélectionnée parmi
I poly-(n-butyl-acrylate) PBA,
II copolymère butadiène-styrène,
et dans laquelle la répartition de la phase élastique dans le composant pulvérulent suit une des possibilités a à c, à savoir
a) coeur élastique dans une coque solide (particules coeur-coque) ; ou
b) plusieurs coeurs élastiques dans une matrice solide ; ou
c) particules coeur-coque de a) réparties dans une matrice solide,
dans laquelle la phase élastique a un diamètre compris entre 10 nm et 100 µm,
dans le composant B d'un matériau de base de prothèse blcomposant autopolymérisant constitué de
A) un composant monomère fluide,
B) un composant pulvérulent contenant des charges,
C) un système d'initiateurs, dans lequel le système d'initiateurs est une combinaison d'acides barbituriques en liaison avec des ions de cuivre et de chlorure et des péroxydes benzoyle ou lauryl,
pour l'obtention d'une ténacité à la rupture ≥ 2 MPa m^{½} et d'une résilience ≥ 900 J/m² dans un plastique choc pour prosthèse.
